# EUROPEAN PATENT APPLICATION

(11) **EP 1 016 417 A1**
(43) Date of publication of application: **05.07.2000**
(21) Application number: 98911070.5
(22) Date of filing: 27.03.1998
(51) Int. Cl.: A61K 47/30, A61K 9/70

(54) **PRESSURE-SENSITIVE ADHESIVES FOR PERCUTANEOUS PLASTERS AND PERCUTANEOUS PLASTERS**

(30) Priority: 07.04.1997 JP 10244797
(71) Applicant: HISAMITSU PHARMACEUTICAL CO. INC., Tosu-shi Saga 841-0017 (JP)
(72) Inventor: HIGO, Naruto, Hisamitsu Pharmaceutical Co.,Inc., Tsukuba-shi, Ibaraki 305-0856 (JP); HIRANO, Munehiko, Hisamitsu Pharmaceutical Co.,Inc, Tsukuba-shi, Ibaraki 305-0856 (JP); SHINMURA, Miyuki, Hisamitsu Pharmaceutical Co.,Inc, Tsukuba-shi, Ibaraki 305-0856 (JP)
(74) Representative: Modiano, Guido, Dr.-Ing.
(86) International application number: JP9801409
(87) International publication number: WO9844949

(57) **Abstract**

This invention offers an adhesive for a percutaneously absorbable patch and also a percutaneously absorbable patch which are capable of containing a high concentration of crotamiton. The adhesive contains crotamiton and pirotiodecane as a compatibilizer and, specifically contains 10 to 40% by weight of an elastic block copolymer of an (A-B)ₙ-A type, 10-45% by weight of liquid paraffin, 3 to 10% by weight of crotamiton and 0.3 to 10% by weight of pirotiodecane as a compatibilizer for crotamiton. The patch comprises the adhesive and a drug.

## Description

### Technical Field

This invention relates to an adhesive for a percutaneously absorbable patch, capable of containing crotamiton stably in a high concentration which is used as a solubilizer or absorption accelerator for a drug and also relates to a percutaneously absorbable patch which contains the adhesive and a drug.

### Background Art

Absorption accelerating compounds have been studied which are incorporated into a percutaneously absorbable patch in expectation of the effects as an absorption accelerator or a solubilizer for dissolving a drug contained in the patch.

Among them, crotamiton is particularly contained in a preparation in order to enhance colchicine in percutaneous absorbability and retentivity in the preparation and such a preparation may be used in a dosage form including ointment, gel, cream, liquid or cataplasm. There is a description in Japanese Patent Application Laid-Open Gazette No. Hei 2-258720 (258720/90) that a preparation containing 0.01 to 2% by weight of colchicine and 0.01 to 20% by weight of crotamiton is appropriate. Japanese Pat. Appln. Laid-Open Gazette No. Hei 4-82828 (82828/92) describes that crotamiton is compounded as a solubulizer in a cataplasm containing indomethacin and that the cataplasm thus prepared has a very high release of the drug and can be used with an object of treating intractable diseases which were hardly treated by conventional cataplasms. Moreover, in order to improve hormones such as estradiol and norethisterone acetate in solubility in an adhesive based on a vinyl acetate copolymer and to accelerate the penetration of the hormones into the skin, crotamiton is compounded in the adhesive in Japanese Pat. Appln. Laid-Open Gazette No. Hei 7-309762 (309762/95).

In order to enhance the solubility of the drugs and achieve much more absorption accelerating effect thereon, it is necessary to add crotamiton at a high concentration to an adhesive for a percutaneously absorbable patch containing the drugs. However, crotamiton has a poor compatibility with polymers and plasticizers used in the adhesive, and oozes from the adhesive with a lapse of time if added at a high concentration. As a result, adhesion expected of the adhesive is deteriorated and, further, the drug contained in the adhesive decreases in solubility and percutaneous absorbability.

In view of the above problems, an object of this invention is to offer an adhesive for a percutaneously absorbable patch and a percutaneously absorbable patch which are capable of containing crotamiton in a high concentration.

### Disclosure of the Invention

The present inventors made intensive studies and, as the result of their studies, they have found that the above-mentioned problems can be solved by an adhesive containing crotamiton and pirotiodecane as a compatibilizer (compatibilizing agent) and, particularly, by an adhesive also containing a specific block copolymer as an adhesive ingredient, and have thus achieved this invention. Thus, this invention relates to the following (1) to (4).
(1) An adhesive for a percutaneously absorbable patch, containing crotamiton and pirotiodecane as a compatibilizer.
(2) The adhesive for a percutaneously absorbable patch according to the above (1), comprising 10 to 40% by weight of an elastic block copolymer of an (A-B)ₙ-A type, 10 to 45% by weight of liquid paraffin, 3 to 10% by weight of crotamiton and 0.3 to 10% by weight of pirotiodecane as a compatibilizer for crotamiton, each of the amounts being based upon the total amount of the adhesive.
(3) A percutaneously absorbable patch wherein the base ingredients (adhesive) according to the above (2) contain a drug in an amount of 0.1 to 10% by weight based upon the total amount of the adhesive.
(4) The adhesive for a percutaneously absorbable patch or the percutaneously absorbable patch according to any of the above (1) to (3), wherein pirotiodecane is present in a compounding ratio by weight of 0.1 to 1 based on the weight of crotamiton.

Pirotiodecane to be contained in the adhesive and the patch of this invention is formally called 1-(2-decylthioethyl)-azacyclopentane-2-one (disclosed in Japanese Pat. Appln. Laid-Open Gazette No. Sho 62-164663 (164663/87)) and was developed as a compound excellent in absorption-accelerating action on drugs through the skin and also high in safety. On the other hand, crotamiton contained as a drug solubilizer in a percutaneously absorbable patch has a poor compatibility with polymers and plasticizers contained in an adhesive for the patch and, when crotamiton is contained therein at a high concentration, it will ooze out with a lapse of time whereby the adhesive does not show an expected adhesion and, moreover, solubility and percutaneous absorbability of the drug in the patch are lowered. The present inventors have then unexpectedly found that, when pirotiodecane is used as a compatibilizer in an adhesive containing crotamiton and especially when contained in a specific ratio to crotamiton, crotamiton is improved in compatibility with a polymer, a plasticizer such as liquid paraffin, etc. used in the adhesive and thereby becomes capable of being stably contained in the adhesive in a high concentration.

Pirotiodecane is used in a ratio by weight of usually 0.1 to 1, preferably 0.2 to 0.8, more preferably 0.4 to 0.7, based on the weight of crotamiton. The amount of crotamiton contained in the base (adhesive) is usually 3 to 10% by weight on the basis of the total weight of the adhesive, while that of pirotiodecane contained is usually 0.3 to 10% by weight, preferably 0.9 to 8% by weight, more preferably 1.2 to 7% by weight, on the same basis. If the ratio of pirotiodecane to crotamiton is less than 0.1, crotamiton will not be improved by pirotiodecane in compatibility with the adhesive ingredients and ooze from the adhesive. If the ratio of pirotiodecane to crotamiton is more than 1, the compatibility will not be improved any more and, rather, pirotiodecane itself will ooze out.

In this invention, the (A-B)ₙ-A-based elastic block copolymer used as a base for the adhesive and the patch is a block copolymer composed of a styrene-based block A and a conjugated-diene-based block B, wherein n is an integer of 1 or more. Such a copolymer includes those easily commercially available such as a styrene-butadiene-styrene block copolymer (trade name: Cariflex TR-1101) and styrene-isoprene-styrene block copolymers (trade names: Cariflex TR-1107 and Cariflex TR-1111) which are manufactured by Shell Chemical Co., Ltd.; styrene-isoprene-styrene block copolymers (trade names: JSR 5000 and JSR 5002) manufactured by Nippon Synthetic Rubber Co., Ltd.; and Quintac 3421 manufactured by Nippon Zeon Co., Ltd. and, particularly, an adhesive for a percutaneously absorbable patch containing a styrene-isoprene-styrene block copolymer is preferably used because the adhesive is excellent in adhesion and little irritant to the skin. It is also preferred that the adhesive and the patch of this invention further contain other polymers such as polyisobutylene. The content thereof is usually 0 to 30% by weight, preferably 5 to 20% by weight based upon the total weight of the adhesive.

As a plasticizer for the base for the adhesive and the patch, liquid paraffin is selected in this invention from among known ones in view of its good compatibility with the (A-B)ₙ-A based elastic block copolymer, heat stability and safety for the skin. Incorporation into a preparation of crotamiton to be used in this invention greatly improves a drug in its solubility in the preparation, releasability from the preparation, percutaneous absorbability, etc. However, crotamiton has a poor compatibility with the (A-B)ₙ-A-based elastic block copolymer and with liquid paraffin used as a plasticizer and, therefore, when crotamiton is contained in the preparation at a high concentration, it may be oozed from the preparation after preserved for a long period, causing a decrease in release of the drug or adhesion of the adhesive. The present inventors have found that crotamiton can be allowed to be stably present in the preparation by means of adding pirotiodecane, especially in specific ratios to crotamiton, to the crotamiton-containing preparation as a means for solving the above problem. Until now, there has been neither any example where pirotiodecane is incorporated as a compatibilizer for crotamiton in a preparation which contains crotamiton and is based upon an elastic block copolymer of an (A-B)ₙ-A type nor known literature suggesting such incorporation.

The use of a base which comprises as essential ingredients the (A-B)ₙ-A-based elastic block copolymer, liquid paraffin as a plasticizer, crotamiton and pirotiodecane achieves more enhanced solubility of and percutaneous absorption accelerating effect on a drug as well as compatibility of crotamiton with the adhesive ingredients. These effects are significantly exhibited especially when the adhesive or the patch comprises 10 to 40 parts by weight of the (A-B)ₙ-A-based elastic block copolymer, 10 to 45 parts by weight of liquid paraffin, 3 to 10 parts by weight of crotamiton and 0.3 to 10 parts by weight of pirotiodecane.

There is no particular limitation to the drug that is added to the adhesive for the percutaneously absorbable patch so far as the drug is a physiologically active substance and has percutaneous absorbability. In some cases, two or more drugs may be contained therein. Further, the drug used in this invention may be in a form of a pharmaceutically acceptable inorganic or organic salt or a so-called pro-drug which exhibits a physiological activity after being percutaneously absorbed. Examples of the drug are anti-inflammatory and analgesic agents (such as indomethacin, ketoprofen, diclofenac, ketorolac, felbinac, flurbiprofen, loxoprofen, etodolag and indomethacin farnesil), non-smoking aids (such as nicotine), antiemetic agents (such as haloperidol, timiperone, benperidol, floropamide and fanizon), prostaglandins (such as PGE1, PGF2α, PGE2 and PGI2), anti-vertigo agents (such as difenidol and betahistine), sympathomimetic agents (such as salbutamol sulfate, tulobuterol hydrochloride, procaterol hydrochloride and maputerol hydrochloride), immunomodulators (such as LPSs, auranofin and lobenzarit), polypeptide-based hormones (such as luteinizing-hormone-releasing hormone (LH-RH) and thyrotropin-releasing hormone (TRH)), antiestrogens (such as tamoxifen and fadrozole hydrochloride), other hormones (such as testosterone), coronary vasodilators (such as nitroglycerin, isosorbide nitrate, diltiazem hydrochloride, nifedipine, nicorandil and nitrendipine), local anesthetics (such as lidocaine, benzocaine, procaine hydrochloride and tetracaine), skeleton muscle relaxants (such as eperisone, tizanidine, tolperisone, inaperisone, pridinol and dantrolene), antihypertensive agents (such as clonidine, reserpine, guanethidine sulfate, efonidipine, pindolol, bopindolol malonate, captopril and delapril), analgesics (such as morphine, buprenorphine hydrochloride, fentanyl citrate, pendazocine and eptazocine hydrobromide), therapeutic agents for urinary disturbance (such as clenbuterol hydrochloride, osaterone acetate, tiroridine hydrochloride, oxybutynin hydrochloride and flavoxate), antiepileptic agents (such as nitrazepam and meprobamate), anti-parkinsonism agents (such as chlorzoxazone and lepodopa), antiallergic agents (such as tranilast, azelastine, ketotifen, mequitazine, ibudilast, oxatomide and emedastine) and central nervous acting agents (such as chlorpromazine, nitrazepam, diazepam, reserpine and imipramine) as well as follicular hormones (such as mestranol, equilin, estradiol, estrogen of a binding type and estriol) and derivatives thereof (such as equilenin, estradiol propionate, estradiol benzoate, ethynyl estradiol, estradiol valerate, estriol propionate, estriol tripropionate and estriol benzoate acetate). The drugs applicable to this invention also include corpus luteal hormones such as progesterone, hydroxyprogesterone caproate, medroxyprogesterone acetate, dydrogesterone, chlormadinone acetate, ethisterone, dimethisterone, norethisterone, norethisterone acetate, norethisterone enanthate, ethinodiol acetate, megestrol acetate and allylestrenol as well as medically acceptable salts thereof. The content of the drug in the patch of this invention is preferably 0.1 to 10% by weight of the total weight of the adhesive.

The percutaneously absorbable preparation and the adhesive of this invention may appropriately contain an appropriate amount of already-known additives in addition to the above-mentioned essential ingredients. Such additives include tackifiers such as polyterpene resins (trade name: YS-Resin manufactured by Yasuhara Chemical Co., Ltd.; trade name: Piccolyte manufactured by Hercules Co., Ltd.), terpene phenol resins (trade name: YS-Polyster manufactured by Yasuhara Chemical Co., Ltd.; trade name: Piccofine manufactured by Hercules Co., Ltd.), petroleum type resins (trade name: Quinton manufactured by Nippon Zeon KK; trade name: Arkon manufactured by Arakawa Chemical Co., Ltd.; trade name: Regalets manufactured by Hercules Co., Ltd.; trade name: Escorez manufactured by Exxon Co., Ltd.; trade name: Wingtac manufactured by Goodyear Co., Ltd.) and rosin resins (trade name: KE-311 manufactured by Arakawa Chemical Go., Ltd.) and water-absorbable polymers such as metal salts of polyacrylic acid, carboxymethylated polyvinyl alcohol, carboxymethyl cellulose and metal salts thereof, a product prepared by introducing a light cross-linkage into carboxymethyl polymer or the like and metal salts of graft saponified product of starch acrylonitrile as well as softeners (such as higher fatty acid, liquid rubber and mineral oil) and inorganic fillers (such as silica, titanium oxide, calcium carbonate and aluminum silicate).

The support for the preparation of this invention is preferably one which has no influence on the release of the drug and has an excellent flexibility, and is preferably selected from a film or woven fabric of polyester, polypropylene, polyethylene, ethylene-vinyl acetate, etc. and an aluminum foil, or a flexible composite material such as a laminate of those plastic films or woven fabrics.

Some methods for producing the percutaneously absorbable patch and the adhesive for the patch according to this invention will be described hereunder. First, the base ingredients such as the (A-B)ₙ-A type elastic block copolymer and liquid paraffin are heat melted and then incorporated with crotamiton and pirotiodecane (and a drug) to give a preparation or an adhesive. This preparation is spread over the above-mentioned support, covered with a liner and cut into a desired shape to give a patch. Alternatively, the preparation may be once spread over a film which has been treated to be made releasable, and then pressed onto a suitable support to transfer the preparation onto the support, and suitably cut to give a patch product. It is also possible that all the ingredients are dissolved in an organic solvent such as hexane, toluene or ethyl acetate, the solution is spread over the above support, the organic solvent is removed therefrom and the resulting preparation is covered with a liner and cut into a desired shape to give a patch or that the above-mentioned solution is once spread over a film which has been made releasable, the organic solvent is removed therefrom and the residue is transferred to a suitable support by pressing and appropriately cut to give a patch.

The adhesive for a percutaneously absorbable patch and the percutaneously absorbable preparation containing the adhesive prepared as above contain a high concentration of crotamiton. Accordingly, they are excellent in solubility and promotion of percutaneously absorption of the drug and also have the effects of enhancing dissolution stability, physical properties and bioavailability of the drug in the preparation upon a long period of preservation.

### Brief Explanation of the Drawings

Fig. 1 shows the results of a hairless mouse skin permeation test on the patches prepared by Examples of this invention and Comparative Examples.

### Best Mode for Carrying Out the Invention

This invention will now be described in more detail by way of the following Examples and Test Examples in which every "part(s)" stands for that/those by weight.

### Example 1

| | |
|---|---|
| Styrene-isoprene-styrene block copolymer (trade name: Cariflex TR-1107) | 40 parts |
| Liquid paraffin | 32 parts |
| Crotamiton | 3 parts |
| Pirotiodecane | 1 part |
| Polyisobutylene | 4 parts |
| Petroleum resin (trade name: Arkon) | 20 parts |

In accordance with this formulation, an adhesive for a percutaneously absorbable patch of this invention was produced by the above-mentioned producing method.

### Example 2

| | |
|---|---|
| Styrene-isoprene-styrene block copolymer (trade name: Cariflex TR-1111) | 30 parts |
| Liquid paraffin | 27 parts |
| Crotamiton | 5 parts |
| Pirotiodecane | 5 parts |
| Polyisobutylene | 8 parts |
| Rosin resin (trade name: KE-311) | 25 parts |

In accordance with this formulation, an adhesive for a percutaneously absorbable patch of this invention was produced by the above-mentioned producing method.

### Example 3

| | |
|---|---|
| Styrene-isoprene-styrene block copolymer (trade name: Cariflex TR-1107) | 30 parts |
| Liquid paraffin | 20 parts |
| Crotamiton | 10 parts |
| Pirotiodecane | 10 parts |
| Petroleum resin (trade name: Escorez) | 30 parts |

In accordance with this formulation, an adhesive for a percutaneously absorbable patch of this invention was produced by the above-mentioned producing method.

### Example 4

| | |
|---|---|
| Styrene-isoprene-styrene block copolymer (trade name: Cariflex TR-1111) | 30 parts |
| Liquid paraffin | 10 parts |
| Crotamiton | 10 parts |
| Pirotiodecane | 5 parts |
| Polyisobutylene | 10 parts |
| Polyterpene resin (trade name: Piccolyte) | 35 parts |

In accordance with this formulation, an adhesive for a percutaneously absorbable patch of this invention was produced by the above-mentioned producing method.

### Example 5

| | |
|---|---|
| Styrene-isoprene-styrene block copolymer (trade name: Cariflex TR-1107) | 30 parts |
| Liquid paraffin | 14 parts |
| Crotamiton | 10 parts |
| Pirotiodecane | 1 part |
| Polyisobutylene | 10 parts |
| Terpene phenol resin (trade name: Piccofine) | 35 parts |

In accordance with this formulation, an adhesive for a percutaneously absorbable patch of this invention was produced by the above-mentioned producing method.

### Example 6

| | |
|---|---|
| Styrene-isoprene-styrene block copolymer (trade name: Cariflex TR-1111) | 30 parts |
| Liquid paraffin | 13 parts |
| Crotamiton | 7 parts |
| Pirotiodecane | 5 parts |
| Polyisobutylene | 10 parts |
| Rosin resin (trade name: KE-311) | 35 parts |

In accordance with this formulation, an adhesive for a percutaneously absorbable patch of this invention was produced by the above-mentioned producing method.

### Example 7

| | |
|---|---|
| Styrene-isoprene-styrene block copolymer (trade name: Cariflex TR-1107) | 30 parts |
| Liquid paraffin | 15 parts |
| Crotamiton | 7 parts |
| Pirotiodecane | 3 parts |
| Polyisobutylene | 10 parts |
| Petroleum resin (trade name: Regalets) | 35 parts |

In accordance with this formulation, an adhesive for a percutaneously absorbable patch of this invention was produced by the above-mentioned producing method.

### Example 8

| | |
|---|---|
| Styrene-isoprene-styrene block copolymer (trade name: Cariflex TR-1111) | 25 parts |
| Liquid paraffin | 32 parts |
| Crotamiton | 5 parts |
| Pirotiodecane | 5 parts |
| Polyisobutylene | 8 parts |
| Petroleum resin (trade name: Arkon) | 25 parts |

In accordance with this formulation, an adhesive for a percutaneously absorbable patch of this invention was produced by the above-mentioned producing method.

### Example 9

| | |
|---|---|
| Styrene-isoprene-styrene block copolymer (trade name: Cariflex TR-1107) | 10 parts |
| Liquid paraffin | 45 parts |
| Crotamiton | 7 parts |
| Pirotiodecane | 5 parts |
| Polyisobutylene | 8 parts |
| Petroleum resin (trade name: Wingtac) | 25 parts |

In accordance with this formulation, an adhesive for a percutaneously absorbable patch of this invention was produced by the above-mentioned producing method.

### Example 10

| | |
|---|---|
| Styrene-isoprene-styrene block copolymer (trade name: Cariflex TR-1111) | 30 parts |
| Liquid paraffin | 13 parts |
| Crotamiton | 6 parts |
| Pirotiodecane | 3 parts |
| Polyisobutylene | 10 parts |
| Rosin resin (trade name: KE-311) | 35 parts |
| Ketoprofen | 3 parts |

In accordance with this formulation, a percutaneously absorbable patch of this invention was produced by the above-mentioned producing method.

### Example 11

| | |
|---|---|
| Styrene-isoprene-styrene block copolymer (trade name: Cariflex TR-1107) | 30 parts |
| Liquid paraffin | 22 parts |
| Crotamiton | 7 parts |
| Pirotiodecane | 3 parts |
| Rosin resin (trade name: KE-311) | 35 parts |
| Eperisone | 1 part |
| Loxoprofen | 2 parts |

In accordance with this formulation, a percutaneously absorbable patch of this invention was produced by the above-mentioned producing method.

### Example 12

| | |
|---|---|
| Styrene-isoprene-styrene block copolymer (trade name: Cariflex TR-1111) | 30 parts |
| Liquid paraffin | 15 parts |
| Crotamiton | 5 parts |
| Pirotiodecane | 2 parts |
| Polyisobutylene | 10 parts |
| Rosin resin (trade name: KE-311) | 35 parts |
| Azelastine | 3 parts |

In accordance with this formulation, a percutaneously absorbable patch of this invention was produced by the above-mentioned producing method.

### Example 13

| | |
|---|---|
| Styrene-isoprene-styrene block copolymer (trade name: Cariflex TR-1107) | 30 parts |
| Liquid paraffin | 14 parts |
| Crotamiton | 5 parts |
| Pirotiodecane | 3 parts |
| Polyisobutylene | 10 parts |
| Rosin resin (trade name: KE-311) | 35 parts |
| Buprenorphine hydrochloride | 3 parts |

In accordance with this formulation, a percutaneously absorbable patch of this invention was produced by the above-mentioned producing method.

### Example 14

| | |
|---|---|
| Styrene-isoprene-styrene block copolymer (trade name: Cariflex TR-1111) | 30 parts |
| Liquid paraffin | 13 parts |
| Crotamiton | 7 parts |
| Pirotiodecane | 2 parts |
| Polyisobutylene | 10 parts |
| Rosin resin (trade name: KE-311) | 35 parts |
| Diazepam | 3 parts |

In accordance with this formulation, a percutaneously absorbable patch of this invention was produced by the above-mentioned producing method.

### Example 15

| | |
|---|---|
| Styrene-isoprene-styrene block copolymer (trade name: Cariflex TR-1107) | 30 parts |
| Liquid paraffin | 12 parts |
| Crotamiton | 5 parts |
| Pirotiodecane | 5 parts |
| Polyisobutylene | 10 parts |
| Rosin resin (trade name: KE-311) | 35 parts |
| Estradiol | 1 part |
| Norethisterone acetate | 2 parts |

In accordance with this formulation, a percutaneously absorbable patch of this invention was produced by the above-mentioned producing method.

### Comparative Example 1

| | |
|---|---|
| Styrene-isoprene-styrene block copolymer (trade name: Cariflex TR-1111) | 30 parts |
| Liquid paraffin | 17.5 parts |
| Crotamiton | 7 parts |
| Pirotiodecane | 0.5 parts |
| Polyisobutylene | 10 parts |
| Rosin resin (trade name: KE-311) | 35 parts |

In accordance with this formulation, an adhesive for a percutaneously absorbable patch was produced by the above-mentioned producing method.

### Comparative Example 2

| | |
|---|---|
| Styrene-isoprene-styrene block copolymer (trade name: Cariflex TR-1107) | 30 parts |
| Liquid paraffin | 8 parts |
| Crotamiton | 7 parts |
| Pirotiodecane | 10 parts |
| Polyisobutylene | 10 parts |
| Rosin resin (trade name: KE-311) | 35 parts |

In accordance with this formulation, an adhesive for a percutaneously absorbable patch was produced by the above-mentioned producing method.

### Comparative Example 3

| | |
|---|---|
| Styrene-isoprene-styrene block copolymer (trade name: Cariflex TR-1111) | 30 parts |
| Liquid paraffin | 15 parts |
| Crotamiton | 7 parts |
| Polyisobutylene | 10 parts |
| Rosin resin (trade name: KE-311) | 35 parts |
| Estradiol | 1 part |
| Norethisterone acetate | 2 parts |

In accordance with this formulation, a percutaneously absorbable patch was produced by the above-mentioned producing method.

### Comparative Example 4

| | |
|---|---|
| Styrene-isoprene-styrene block copolymer (trade name: Cariflex TR-1107) | 30 parts |
| Liquid paraffin | 19 parts |
| Pirotiodecane | 3 parts |
| Polyisobutylene | 10 parts |
| Rosin resin (trade name: KE-311) | 35 parts |
| Estradiol | 1 part |
| Norethisterone acetate | 2 parts |

In accordance with this formulation, a percutaneously absorbable patch was produced by the above-mentioned producing method.

### Comparative Example 5

| | |
|---|---|
| Styrene-isoprene-styrene block copolymer (trade name: Cariflex TR-1111) | 30 parts |
| Liquid paraffin | 22 parts |
| Polyisobutylene | 10 parts |
| Rosin resin (trade name: KE-311) | 35 parts |
| Estradiol | 1 part |
| Norethisterone acetate | 2 parts |

In accordance with this formulation, a percutaneously absorbable patch was produced by the above-mentioned producing method.

### Test Example 1 (Compatibility Test)

A compatibility test was performed only on the oily ingredients (the ingredients other than estradiol and norethisterone acetate) in the formulation of Example 15. More particularly, in a glass sample tube, the ratio by weight of pirotiodecane to crotamiton was changed within 0, 0.05, 0.1, 0.2, 0.3, 0.4, 0.5, 0.6, 0.7, 0.8, 0.9, 1, 1.2 and 1.4 while the compounding ratio of liquid, paraffin to crotamiton in Example 15 was maintained. After stirred, the samples were observed for compatibility and the results are given in Table 1. The samples with the compounding ratio of pirotiodecane being 0 and 0.05 were observed to be turbid with crotamiton. When the ratio was 0.1 and 1, crotamiton was almost compatible with other ingredients although some turbidity was noted. When the ratio became 1.2 or more, pirotiodecane was separated out. From the results, it is noted that, when pirotiodecane is used in the crotamiton-containing adhesive in the specific ratios to crotamiton, crotamiton is enhanced in compatibility with the adhesive ingredients and improved in stability in the adhesive base. This supports the usefulness of the adhesive for a percutaneously absorbable patch according to this invention.

**Table 1**

| Compounding Ratio | Resulting Appearance |
|---|---|
| 0 | turbid |
| 0.05 | turbid |
| 0.1 | somewhat turbid |
| 0.2 | transparent |
| 0.3 | transparent |
| 0.4 | transparent |
| 0.5 | transparent |
| 0.6 | transparent |
| 0.7 | transparent |
| 0.8 | transparent |
| 0.9 | transparent |
| 1.0 | somewhat turbid |
| 1.2 | separated and turbid |
| 1.4 | separated and turbid |

### Test Example 2 (Adhesion Test)

The percutaneously absorbable patches and adhesives of Examples 10, 13 and 15 and Comparative Examples 1 and 2 (where the ratios by weight of pirotiodecane to crotamiton were 0.5, 0.6, 1, 0.07 and 1.4, respectively) were punched out into circular pieces 3 cm in diameter, placed in a packaging material sealed with a composite film of aluminum and polyethylene and preserved at 40°C for four weeks and, thereafter, a probe tack test was carried out. In this test, each sample with the adhesive side made downward was adhered down to a sample-holding ring so that the adhesive side and the probe made of stainless steel were contacted together and, then the sample was measured for the force required for separating the probe from the adhesive side of the sample. The results are given in Table 2 (unit: grams). Incidentally, when the liner was peeled off the patch and the adhesive in starting the probe tack test, no particular inconvenience was noted in Examples 10, 13 and 15 while oozing of crotamiton was seen in Comparative Example 1 and oozing of pirotiodecane was seen in Comparative Example 2. From those results, it is noted that, when pirotiodecane is used in specific ratios to crotamiton in the crotamiton-containing patch, crotamiton is enhanced in compatibility with the other adhesive base ingredients and improved in stability in the adhesive base. Thus, this supports the usefulness of the adhesive for a percutaneously absorbable patch according to this invention.

**Table 2**

| | Appearance | 1st Run | 2nd Run | 3rd Run | 4th Run | 5th Run | Average | S.D. |
|---|---|---|---|---|---|---|---|---|
| Ex. 10 | No oozing out | 300 | 310 | 296 | 304 | 315 | 305.0 | 7.6 |
| Ex. 13 | No oozing out | 310 | 314 | 301 | 318 | 304 | 309.4 | 7.0 |
| Ex. 15 | No oozing out | 308 | 299 | 310 | 318 | 304 | 307.8 | 7.1 |
| Comp. Ex.1 | Crotamiton oozed out | 168 | 150 | 162 | 159 | 149 | 157.6 | 8.1 |
| Comp. Ex.2 | Pirotiodecane oozed out | 170 | 158 | 165 | 176 | 172 | 168.2 | 6.9 |

### Test Example 3 (Percutaneous Permeability Test in Hairless Mice)

The patches of Example 15 and Comparative Examples 3-5 (Comparative Example 3 is the patch of Example 15 with pirotiodecane removed; Comparative Example 4 is the patch of Example 15 with crotamiton removed; Comparative Example 5 is the patch of Example 15 with both crotamiton and pirotiodecane removed) were each punched out into a circle 3 cm in diameter and subjected to a percutaneous permeability test using hairless mice. Another circular patch 3 cm in diameter from each patch was placed in a packaging material sealed with a composite film of aluminum and polyethylene and preserved at 40°C for four weeks and, thereafter, subjected to an observation of crystallization and oozing out and also to a percutaneous permeation experiment using hairless mice again. The results were compared with the initial ones. Each percutaneous permeation experiment using hairless mice was repeated three times to indicate the average value ± S. D. of the drug permeating speeds in a steady state (unit: mg/cm²). The results are shown in Table 3 and Fig. 1, which show that the patches of Comparative Example 4 and Comparative 5 containing no crotamiton were observed to have the drug crystallized therein and were also low in the drug-permeating levels in the percutaneous permeation experiment using hairless mice. Example 15 containing the combination of crotamiton and pirotiodecane and Comparative Example 3 containing only crotamiton were not observed to have the drug crystallized therein and were high in the initial levels in the percutaneous permeation experiment using hairless mice. Comparative Example 3 containing only crotamiton was, however, observed to ooze crotamiton out during the preservation and was lowered in the levels after the preservation in the percutaneous permeation experiment using hairless mice. From the above results, it is apparent that the patch of Example 15 exhibited the high permeability of the drug through the hairless mouse skin and no oozing out of crotamiton because the crotamiton-containing patch also contained pirotiodecane. Thus, the results support the usefulness of the percutaneous absorbable patch and of the adhesive therefor of this invention.

**Table 3**

| | Percutaneous Permeation Test on Hairless Mice | | Appearance after 4 weeks at 40°C |
|---|---|---|---|
| | Initial | after 4 weeks at 40°C | |
| Example 15 | 0.085 ± 0.011 | 0.084 ± 0.007 | Neither oozed out nor crystallized |
| Comp.Ex. 3 | 0.080 ± 0.016 | 0.043 ± 0.010 | Oozed out |
| Comp.Ex. 4 | 0.054 ± 0.012 | 0.018 ± 0.004 | Crystallized |
| Comp.Ex. 5 | 0.041 ± 0.009 | 0.010 ± 0.003 | Crystallized |

### Industrial Applicability

As illustrated hereinabove, the adhesive for a percutaneously absorbable patch of this invention containing an (A-B)ₙ-A-based elastic block copolymer, liquid paraffin, crotamiton and pirotiodecane as essential ingredients and the percutaneously absorbable patch of this invention comprising the adhesive and a drug enable a high concentration of crotamiton to be stably compatible with the adhesive ingredients and are thereby capable of containing a drug in a high concentration and can be expected to exert an much greater absorption accelerating effect on the drug whereby this invention is quite useful in industries.

## Claims

1. An adhesive for a percutaneously absorbable patch, containing crotamiton and pirotiodecane as a compatibilizer.

2. The adhesive for a percutaneously absorbable patch according to claim 1, comprising 10 to 40% by weight of an (A-B)ₙ-A-based elastic block copolymer, 10 to 45% by weight of liquid paraffin, 3 to 10% by weight of crotamiton and 0.3 to 10% by weight of pirotiodecane as a compatibilizer for crotamiton, each of the amounts being based upon the total amount of the adhesive.

3. A percutaneously absorbable patch comprising the adhesive according to claim 2 having a drug in an amount of 0.1 to 10% by weight based upon the total amount of the adhesive contained therein.

4. The adhesive for a percutaneously absorbable patch or the percutaneously absorbable patch according to any of claims 1 to 3, wherein pirotiodecane is present in a compounding ratio by weight of 0.1 to 1 based on the weight of crotamiton.
